# EUROPEAN PATENT SPECIFICATION

(11) **EP 1 990 405 B1**
(45) Date of publication and mention of the grant of the patent: **26.07.2017**
(21) Application number: 07009217.6
(22) Date of filing: 08.05.2007
(51) Int. Cl.: C12N 1/21, C12P 19/56, C12R 1/46

(54) **Genetically modified strains producing anthracycline metabolites useful as cancer drugs**
Genetisch modifizierte Stämme zur Produktion von Anthracyclin-Metaboliten, die als Krebsheilmittel dienen können
Souches modifiées génétiquement produisant des métabolites anthracyclines utiles en tant que médicaments contre le cancer

(43) Date of publication of application: 12.11.2008
(73) Proprietor: Provivo Oy, 04601 Mäntsälä (FI)
(72) Inventor: Lambert, Michael, Dr., 63584 Gründau (DE); Ylihonko, Kristiina, Dr., 20781 Kaarina (FI)
(74) Representative: Berggren Oy, Turku

(56) References cited:
- WO-A-2006/111561
- MADDURI K ET AL: "Production of the antitumor drug epirubicin (4'-epidoxorubicin) and its precursor by a genetically engineered strain of Streptomyces peucetius" NATURE BIOTECHNOLOGY, NATURE PUB. CO, NEW YORK, NY, US, vol. 16, no. 1, January 1998 (1998-01), pages 69-74, XP002210929 ISSN: 1087-0156
- JANSSON A ET AL: "Crystal Structure of Aclacinomycin-10-Hydroxylase, a S-Adenosyl-l-Methionine-dependent Methyltransferase Homolog Involved in Anthracycline Biosynthesis in Streptomyces purpurascens" JOURNAL OF MOLECULAR BIOLOGY, LONDON, GB, vol. 334, no. 2, 21 November 2003 (2003-11-21), pages 269-280, XP004470974 ISSN: 0022-2836
- HAUTALA ANNE ET AL: "Studies on a second and third ring cyclization in anthracycline biosynthesis." THE JOURNAL OF ANTIBIOTICS FEB 2003, vol. 56, no. 2, February 2003 (2003-02), pages 143-153, XP009088730 ISSN: 0021-8820
- WANG YULONG ET AL: "Modification of aklavinone and aclacinomycins in vitro and in vivo by rhodomycin biosynthesis gene products." FEMS MICROBIOLOGY LETTERS 19 FEB 2002, vol. 208, no. 1, 19 February 2002 (2002-02-19), pages 117-122, XP002463346 ISSN: 0378-1097
- KUNNARI T J ET AL: "Hybrid anthracyclines from a genetically engineered Streptomyces galilaeus mutant." THE JOURNAL OF ORGANIC CHEMISTRY 19 MAY 2000, vol. 65, no. 10, 19 May 2000 (2000-05-19), pages 2851-2855, XP002463347 ISSN: 0022-3263
- LOMOVSKAYA NATALIE ET AL: "Doxorubicin overproduction in Streptomyces peucetius: Cloning and characterization of the dnrU ketoreductase and dnrV genes and the doxA cytochrome P-450 hydroxylase gene" JOURNAL OF BACTERIOLOGY, vol. 181, no. 1, January 1999 (1999-01), pages 305-318, XP002342780 ISSN: 0021-9193
- SEGURA D ET AL: "Anthracyclines: Isolation of overproducing strains by the selection and genetic recombination of putative regulatory mutants of Streptomyces peucetius var. caesius" APPLIED MICROBIOLOGY AND BIOTECHNOLOGY, vol. 48, no. 5, November 1997 (1997-11), pages 615-620, XP002463348 ISSN: 0175-7598

## Description

### Field of the invention

The present invention relates to genetically modified strains of *Streptomyces peucetius* and to the use of such strains in the production of high titre mixtures of anthracycline metabolites, such as 4'-epidaunorubicin, 13-dihydro-epidaunorubicin, 4'-epi-feudomycin and ε-rhodomycinone by single batch fermentation, useful in downstream processes according to the present invention for producing the active pharmaceutical ingredients epirubicin and/or idarubicin.

### Background of the invention

Daunomycins are a group of antitumor antibiotics produced by several *Streptomyces sp.*, such as *S. peucetius, S. coerulorubidus*, *S. griseus*, *Streptomyces sp. C5*, *S. peucetius var. caesius*, and *S. bifurcus.* The basic compound of the group is daunomycin (DiMarco et al., 1964).

Daunomycin and its 14-hydroxy derivative doxorubicin have been used in cancer chemotherapy since 1967 and 1971, respectively. Daunomycin is particularly used for hematologic malignancies, whereas doxorubicin exhibits a broad anticancer spectrum. Considering all the known anthracyclines currently in clinical use, six out of seven are members of daunomycin group. Manufacturing processes for these compounds use daunorubicin or its aglycone, daunomycinone, as the starting material for chemical synthesis.

Anthracyclines have been in use for cancer chemotherapy for more than thirty years. Doxorubicin is still today the mainly used cytotoxic drug because of its extraordinarily wide spectrum of malignancies. Epirubicin, the second most important antitumour antibiotic soon after doxorubicin, is becoming even more important.

Daunomycins may be described by the general formula I 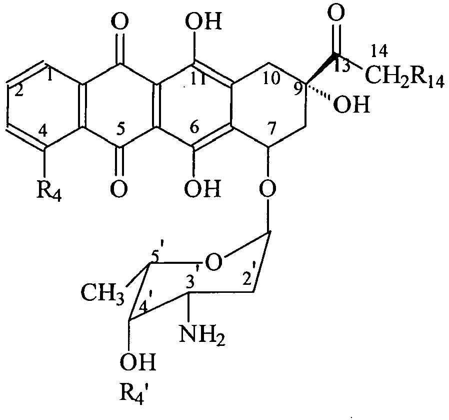 and its most important derivatives are shown in Table 1.

**Table 1**

| | R₄ | R₁₄ | 2'substituent | 4' |
|---|---|---|---|---|
| Daunorubicin | OCH₃ | H | | |
| Doxorubicin | OCH₃ | OH | | |
| Epirubicin | OCH₃ | OH | | 4'epi isomer |
| Idarubicin | H | H | | |
| Annamycin | H | OH | I | 4'epi isomer |
| Pirarubicin | OCH₃ | OH | | 4'-O-tetrahydropyranyl |
| Valrubicin | OCH₃ | valerate | | trifluoroacetyl group |

Epirubicin is clinically used for many types of cancer. The market is growing as it competes with doxorubicin. New formulations, conjugates and new combinations with other cancer drugs also expand the usage of epirubicin. Epirubicin is manufactured by a process which comprises producing daunorubicin by fermentation and synthetically modifying the aglycone and sugar moiety, disclosed e.g. in US Patent 5,874,550.

Idarubicin (4-demethoxy-daunorubicin) is used to treat certain types of cancer, including leukemia, lymphoma, and other diseases of the bone marrow. It is claimed to cause less side effects than doxorubicin. The global annual market for idarubicin is, however, no more than 20 kg, presumably because of its exceptionally high price, which is due to a very complicated manufacturing process. Idarubicinone is manufactured starting from daunomycinone, obtained from fermentation of daunorubicin with subsequent acidic hydrolysis. Daunorubicinone is further synthetically modified to idarubicinone. A sugar residue, daunosamine, is attached by a complicated synthetic reaction series, as described in e.g. US Patent No. 4,325,946.

Prior to genetic engineering, generation of strains producing desired non-endogenous substances was almost impossible. Even though the modifications in the chemical structure are minor, e.g. epidaunorubicin differs from daunorubicin only in the stereochemistry of the 4' OH-group in the daunosamine moiety; several mutations are needed to cause the alterations. This means that several rounds of mutagenesis are needed and million of clones need to be selected and tested in order to find a desired change in a chemical structure.

Today it is well known that genetic modification of bacterial strains facilitates the production of non-endogenous metabolites. However, it is also well known that these strains accumulate the foreign metabolites, hybrid compounds, in poor quantities even though endogenous metabolites are produced in relatively high level. This fact results in poor economic and the processes are often not commercially feasible.

European patent publication EP1123310 describes a successful modification of the sugar moiety of anthracyclines by introducing the gene *snog*C to an anthracycline producing strain resulting in formation of aklavinone-4'-epi-2-deoxyfucose. However, the production titres remain low as discussed above.

Furthermore, purification of a desired metabolite from the complex matrix comprising typically 10-20 similar products is often not successful. Yet another reason for low yields is a poor resistance against non-endogenous compounds by the producer strains. Adaptation of the producer strain to a non-endogenous metabolite by step-wise addition of a toxic product, such as epidaunorubicin, epirubicin or idarubicin to culture broth is time consuming and preferably results in accumulation of a metabolite that is less toxic to the producer strain.

Recently some progress regarding yield and economy of the biotechnological production of the anthracycline epirubicin has been reported, for example in International Patent Publication WO 2006/111561 A1. By mutagenization of a strain from the species *Streptomyces peucetius* a microbial strain was obtained, which is producing epidaunorubicin (a precursor of epirubicin) and/or epirubicin itself at concentrations of at least 0.1 g/l culture broth.

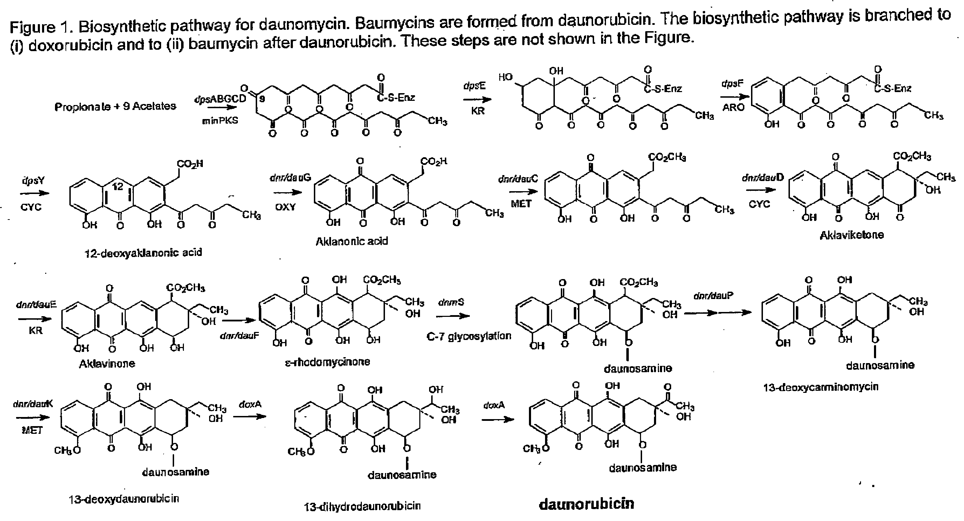

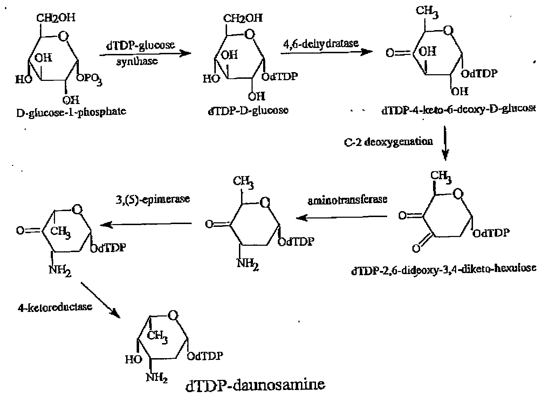

The biosynthetic pathway for anthracyclines and substrate specificities are described in several publications (for a recent review see Niemi et al., 2002 or K. Madduri, Nature Biotechnology, Nature pub. co, New York, NY, US vol. No. 1, January 1988, pages 69-74) even though the final steps of daunomycin biosynthesis, the reactions modifying the aglycone moiety after glycosylation and the molecular genetics therein are not fully understood. The biosynthetic pathway is shown in Figure 1.

The triumph of anthracyclines seems to be continuous. Several new formulations, conjugates, new molecules and prodrugs of daunomycin class are in the pipelines for cancer drugs. Anthracycline production starting from daunorubicin by chemical synthesis is a multi-step process that results in relatively low yields suggesting that improved processes are needed. Thus, there is a well recognized need for improved producer strains and efficient, commercially viable productions processes.

### BRIEF DESCRIPTION OF THE DRAWINGS

Figure 1 is a schematic presentation of the biosynthetic pathway for daunomycin class of anthracyclines.
Figure 2 is a schematic presentation of a process for producing and isolating crude epidaunomycins and ε-rhodomycinone from a fermentation batch.
Figure 3 shows a typical chromatogram identifying the metabolites obtained from a microbial strain with capabilities for high titre production of epidaunomycins and ε-rhodomycinone relating to the present invention. Typical metabolite profile: Rt=6.950 13-DHED, Rt=7.850 4'-epi-feudomycin, Rt=8.725 4'-Epidaunorubicin, Rt=15.842 ε-rhodomycinone

### BRIEF DESCRIPTION OF THE INVENTION

Throughout this description epidaunomycins, such as epidaunorubicin (CAS # 56390-08-0), 13-dihydro-epidaunorubicin = 4'-epi-daunorubicinol (referred to as 13-DHED hereinafter), and epi-feudomycin (designated as analogously to Feudomycin B) are collectively referred to as epidaunomycins.

The present invention relates to improved, genetically modified producer strains, preferably strains derived from *Streptomyces peucetius,* which produce a mixture of antracycline metabolites, including non-endogeneous metabolites, such as epidaunomycins, at a titre of at least 0.5 g per litre culture broth, and which produce at least 0.1 g/l fermentation broth of each of the compounds epidaunorubicin, 13-dihydro-epidaunorubicin, 4'-epi-feudomycin and ε-rhodomycinone. These anthracycline metabolites can be used for the subsequent production of anthracycline antibiotics, particularly epirubicin and idarubicin.

In the present work we have used a mutant strain of *S. peucetius var. caesius*, deposited at DSMZ with the deposition No. DSM 12245, which is blocked in biosynthesis of baumycins, and is able to produce daunomycin at a more than hundred times higher titre than the wild type strain. The strain also produces increased amounts of ε-rhodomycinone. The strain was further modified genetically by replacing the functional gene *dnm*V (Otten et al., 1997) by the gene *ekr8* resulting in opposite stereochemistry in the 4'-position of daunomycin metabolites providing thus epidaunomycin metabolites family. The gene used for replacement was surprisingly found in a shotgun cloning experiment related to isolation of genes from bacterial cultures with a probe derived from *snog*C (Torkkell et al., 2001). The genetically modified strain, designated as G001/pB70dv, accumulated epidaunorubin metabolites, roughly 800 mg per 1 litre of culture broth and simultaneously ε-rhodomycinone was produced in relatively high level; 200-300 mg/l.

However, a mixture of epidaunomycin metabolites found contained 4-OH-compounds so called epi-carminomycins making purification process somewhat complicated.

Different gene constructs available were introduced into a strain obtained by dropping away the plasmid pB70dv from the host strain G001, and surprisingly, the plasmid pB89rdmB caused production of epidaunomycin metabolites without epi-carminomycins. Furthermore, unexpected increase in the production titre was obtained when this strain culture was treated once again with a mutagen. 200 mutants were studied in test tube cultivations for production of epidaunomycins and ε-rhodomycinone and high-producing strains producing more than 1 g of anthracycline metabolites per litre of culture broth were identified. These strains produce good mixtures of epidaunomycin metabolites and ε-rhodomycinone: Typically epidaunorubicin:13-DHED:4'-epi-feudomycin :ε-rhodomycinone with titres of 600 mg/l: 500mg/l: 200 mg/l: 200 mg/l, respectively. Minor quantities of epirubicin were also found as side product in all cultivations.

Glycosylation of the aglycone moiety does not proceed completely with these strains and the critical biosynthetic intermediate, ε-rhodomycinone, is found in remarkable quantities in the culture broth. Cultivation of these genetically modified strains using a resin in fermentation broth resulted in even higher titres of metabolites and facilitates the down stream process.

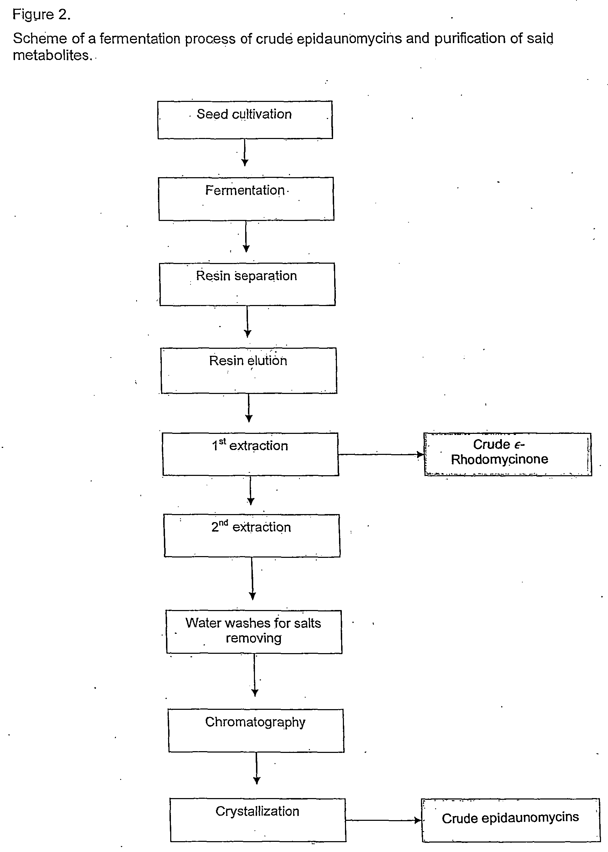

The present invention further relates to a method of producing and isolating crude epidaunomycins, including epidaunorubicin, 13-dihydro-epidaunorubicin (13-DHED), 4'-epi-feudomycin, and ε-rhodomycinone from a single fermentation batch. The process scheme according to the present invention is generally described in Figure 2.

The present invention aims at maximizing the epidaunomycin and ε-rhodomycinone production, minimizing the production of non-endogenous side products and to simplify in this manner the subsequent downstream process. Important aspects of a producer strain suitable for use in the production process of the present invention is
i) that the crude metabolite profile is suitable for down stream processing and;
ii) that the capability of overproduction is not limited by a sensitivity of the strain to epidaunomycins and ε-rhodomycinone.

In the process according to the present invention a single fermentation batch is used as a source of synthetic material for both epirubicin and idarubicin, i.e., both commercially important anthracyclines.

### DETAILED DESCRIPTION OF THE INVENTION

Taking into consideration the known drawback of the prior art, it would be of essential economical interest, to be able to provide in a single batch fermentation a high titre of a mixture of non-endogenous metabolites, suitable for the subsequent manufacturing of more than one anthracycline antibiotic, particularly epirubicin and idarubicin. For simplifying subsequent downstream processings, an even more economical aspect is to avoid the additional formation of endogenous compounds as far as possible. All this is object of the present invention.

Production of one anthracycline, such as epirubicin, by single batch fermentations using optimized microbial strains is a start for getting more efficient production purposes. But the best solution for commercially viable production processes would be to work with modified microbial strains delivering in single batch fermentation at one time a high titre of a mixture of metabolites useful as starting material for the production of more than one anthracycline antibiotic.

The present invention provides improved producer strains for use in a process for producing epidaunomycins and ε-rhodomycinone by fermentation. These strains produce anthracycline metabolites at a titre of at least 0.5 g/l, wherein the strains are producing at least 0.1 g/l fermentation broth of each of the compounds epidaunorubicin, 13-dihydro-epidaunorubicin, 4'-epi-feudomycin and ε-rhodomycinone.

Several *Streptomyces* strains are able to produce daunomycin, whereas *S. peucetius* is preferably used as the producer strain for epidaunomycins in the present invention. Throughout the present description, including the working examples, *S. peucetius var. caesius* G001 (DSM12245) is used as the parent strain which is used for genetic modifications to obtain the strain accumulating epidaunomycins and ε-rhodomycinone.

However, any strain sharing the following characteristics is suitable for this process:
1. Capacity for high production of epidaunomycin metabolites; exceeding 0.1 g/l in total.
2. An incomplete glycosylation system to obtain ε-rhodomycinone in the fermentation process; more than 10 % of the whole anthracycline metabolites fraction.
3. All the metabolites epidaunorubicin, 13-DHED, epi-feudomycin and ε-rhodomycinone are produced in amounts of at least 10 % each of the total anthracycline metabolite fraction.
3. Biosynthetic pathway to produce daunorubicin and related metabolites is blocked.

A mutant strain of *S. peucetius var. caesius* G001 was used as parent strain of this invention. However, several other strains sharing biosynthetic machinery for production of daunomycins are equally suitable. The strain was blocked in production of baumycins by a chemical mutagen, NTG (N-methyl-N'-nitro-N-nitrosoguanidine), and corresponding gene, *dnrH,* was sequenced in order to identify the point mutation resulting in blocking baumycins production. The point mutation caused the change in amino acid; Gly was replaced by Asp. Even though any daunorubicin producer is suitable, a high-producing strain blocked in biosynthesis of baumycins is a preferred host strain for this invention.

Cloning of the genes for 4'-ketoreductase could be done by any known method that results in availability of the gene to be expressed in a selected host. We used a DNA-fragment derived from the gene *snog*C, which has previously been shown to create 4'epi-anthracyclines by Ylihonko et al. (1999), as a probe for screening corresponding ketoreductase genes with a better concomitant action with glycosyl transferases of daunosamine pathway, e.g. ekr8. Using *E. coli* as a host strain for a gene library, hybridization is an advantageous screening strategy. The probe for hybridization may be any fragment derived from *snog*C or similar gene and generated by subcloning with suitable restriction sites or by PCR amplification. Colonies for the gene library are transferred to membranes for filter hybridization, and nylon membranes are typically used. Any method for detection for hybridization may be used but, in particular, the DIG System (Boehringer Mannheim, GmbH, Germany) is useful. Since the probe is heterologous to the hybridized DNA, it is preferable to carry out stringent washes of hybridization at 65°C in a low salt concentration, according to Boehringer Mannheim's manual, DIG System User's Guide for Filter hybridization. The functionality of the gene was demonstrated by experiments to detect epidaunomycins (besides ε-rhodomycinone) in the culture broth by the strain carrying *ekr*8 cloned in a high copy number expression vector, pIJE486. Any 4'-ketoreductase gene that can convert the stereochemistry of the 4'-OH-group in daunosamine is suitable for cloning into a daunorubicin-producing strain to generate production of epidaunomycin (besides ε-rhodomycinone) according to the present invention, but it is preferred that the gene is expressed in high level.

According to the biosynthetic pathway for daunomycins, the gene *dnm*V (Otten et al., 1997) is responsible for the ketoreduction step in daunosamine pathway. This particular gene is a competitor for a transferred gene and is, therefore, preferably inactivated or deleted. Inactivation of the gene *dnm*V may be achieved by any known technique for disrupting a gene function. For this purpose homologous recombination for gene inactivation is preferred. It is also possible to cause a mutation to the gene by random techniques such as chemical mutagenization.

The gene *ekr8* was cloned in different constructs into a strain, obtained by plasmid dropping of parent strain G001, and the metabolites of the achieved strains were analysed. The metabolite profile of one clone was considered promising for down stream purposes. This clone carries the plasmid pB89rdmB (*rdmB*, see Jansson et al. 2003) and produced epidaunomycins and ε-rhodomycinone at a titre corresponding roughly to 50 % of the amount of daunomycins produced by the strain G001. The plasmid pB89rdmB contains the genes *ekr8* and *rdmB.* This clone was designated as G005a. The gene construct could be expressed in any vector capable of replicating in streptomycetes. A high copy number plasmid is, however, preferably used as a vector. For this invention the plasmid vector pIJE486 (Ylihonko et al., 1996) was found to be advantageous.

Several methods for introducing DNA into host cells have been described. A highly preferred procedure is protoplast transformation and it is used thorough this work. However, any method and any vector used to generate a strain having the characteristics of a strain according to the present invention, and described above, is useful.

It was found that the strain mentioned above, containing plasmid pB89rdmB, is somewhat sensitive to epidaunomycins as endogenous resistance against daunomycins was not sufficient and resulted in poor stability of the strain as was noticed by changes in production titres found in repeated cultivations. Increasing the resistance by adaptation with epirubicin or epidaunorubicin resulted in higher titres of 13DHED. The strain culture was treated in stringent conditions with a mutagen and even though any chemical mutagens could be used, NTG is preferred. A clone from mutagenesis treatment with increased production of the same metabolites as compared to the pB89rdmB containing clone was obtained, the stability in a long-term was noticed. The stability is crucial for commercial production and was therefore extremely advantageous even though surprising. The genetically modified strain is stabile without selection pressure.

The new clone achieves the peak of the production level in the eighth day, whereas the sixth day is the best for parent strain G001. Typically the mutant strain produces epidaunorubicin, 13-DHED, epi-feudomycin and ε-rhodomycinone as major products, whereas the rest of the anthracycline fraction covers less than 10% of the yield. Table 2 provides information about typical metabolite profiles of the strains for the presented invention. Quantities are given in mg/l

**Table 2.**

| Strain | 4'-epidaunorubicin | 13-DHED | 4'-epi-feudo-mycin B | Other epidauno-mycins (not specified)* | ε-rhodomycinone |
|---|---|---|---|---|---|
| G001/pB70dv | 250-350 | 50 - 150 | na | 350 - 450 | 200-300 |
| pB89rdmB containing clone | 350 - 450 | 250 - 400 | 150 - 250 | < 100 | 150 - 300 |
| NTG mutagenization | 500-700 | 400 - 600 | 150 - 300 | < 100 | 150 - 300 |

| | | | | | |
|---|---|---|---|---|---|
| * mainly epicarminomycins | | | | | |

A well-known difficulty when attempting to use *S. peucetius* or related strains in the manufacturing of anthracyclines for clinical use is the poor stability, changes in production titre of cultivations of the strains in terms of productivity. It is not economically viable to use such strains and, at its worst, it causes noticeable economic disadvantages and, in the worst cases, leads to the lack of desired cytotoxic drugs for cancer patients. The effect of the instability of the strains to their productivity is a consequence of the mutagenicity of daunorubicin and related metabolites. Therefore, a stable producer strain according to the present invention is advantageous for manufacturing anthracyclines, and a guarantee for continuous supply of important cytotoxic anticancer drugs.

Cultivation of a modified producer strain according to the present invention, preferably a *Streptomyces* strain, most preferably a *Streptomyces peucetius* strain, can be carried out in a medium containing suitable nutrient sources. A preferred medium is the E1 medium, which is described in Experiment 2. The plasmid body of pIJE486 includes a gene responsible for resistance to thiostrepton. Therefore, general experimental protocols would suggest maintaining a selection pressure to keep the plasmid containing strain in the cultivations. Surprisingly, however, cultivations of the said strain in the absence of thiostrepton did not cause loss of the cloned plasmid, suggesting that E1 medium without the supplied antibiotic is preferred in the cultivations.

Addition of an adsorbing resin to a cultivation broth is described in processes for anthracyclines (Torkkell et al. 2001 and Metsä-Ketelä et al., 2003) The toxic product which is also a mutagen affects in two ways; (i) less production caused by toxic effect and (ii) mutagenization of a producer strain. Therefore, an adsorbent which does not interfere with the product or bacteria in cultivation, but adsorbs both the excreted metabolites and those accumulated in cells is preferable.

In a preferred embodiment of the present invention a non-ionic adsorbent is added to the medium before or during the cultivation to collect metabolites from fermentation broth, increasing the recovery per batch. A polystyrene resin, such as Amberlite XAD-7 (CAS # 37380-43-1) (Rohm & Haas Germany GmbH, Frankfurt) or Diaion HP-20 in a suitable medium, such as E1 described in more detail in the experimental section, increases the recovery of epidaunorubicin13-DHED and epi-feudomycin. ε-rhodomycinone is alongside adsorbed from the fermentation broth as well. The higher yield is due to the extremely strong adsorbing ability of the XAD-7 for epidaunomycin metabolites by cross-linking the molecules. Crude epidaunomycins obtained as the yield of recovery contains roughly 500 - 700 mg/l of epidaunorubicin, 400 - 600 mg/l of 13-DHED, 150 - 300 mg/l of 4'-epi-feudomycin and 150 - 300 mg/l of ε-rhodomycinone. If Diaion HP-20 is used as an adsorbent, the same yields are recovered but the amount of adsorbent supplemented to the E1-medium has to be higher significantly.

The fermentation process can be carried out in any conditions enabling the maximum production of epidaunomycins and ε-rhodomycinone. It is, however, advantageous to carry out fermentations in a temperature range of 26°C to 34°C at pH 6.5-7.5 for 7 to 14 days. The adsorbent may be added at any time of cultivation, but the best yield is obtained if the adsorbent is added to the culture in the beginning of cultivation.

The substances adsorbed in the adsorbent resin, comprising mostly epidaunomycins and ε-rhodomycinone, were separated from the culture broth by decanting. Resin was extracted using organic solvents, methanol and butanol being preferred. An amount of solvent as 10-50 % of the volume of the culture broth in a fermentor is recommended. Repeating extraction 1 - 5 times liberates adsorbent used from the all anthracycline metabolites, and resin is re-cyclable after washing with water.

Separation of the aglycone fraction, which contains ε-rhodomycinone, is advantageous after recovery of all metabolites from the adsorbent.

### Further processing of the crude anthracyclines

### Conversion of 13-DHED to epidaunorubicin

13-DHED, is easily separated from glycosidic fraction by chromatography followed by crystallization alongside with epidaunorubicin separation. 13-DHED could be adsorbing to a resin added to the culture broth of *Streptomyces* strain with capabilities for daunomycin synthesis and especially the late steps. Even though any strain is suitable, it is advantageous to use the strain which is blocked in early biosynthetic pathway and unable to accumulate daunomycin metabolites.

### Conversion of epidaunorubicin to epirubicin

Crude epidaunorubicin (purity ≥ 80 %) is used as a starting material for synthetic chemistry to obtain epirubicin, which is a frequently used cancer drug. Any synthetic or biocatalytic reaction series may be used for the 14-hydroxylation.

There are several possibilities to convert epidaunorubicin into its 14-hydroxylated form, epirubicin. The endogeneous gene product alone, 14-hydroxylase, is not sufficiently active in the cultural conditions to convert all epidaunorubicin formed into epirubicin even though minor amounts of epirubicin is found in the culture broth. According to our experiments (data not shown), even high copies of the gene for 14-hydroxylase, failed to complete the process for epirubicin production. Nevertheless, two US patent publications, US 5,955,319 and US 6,210,930 disclose the conversion of daunorubicin into doxorubicin in a low level by the gene product of *doxA.* Apparently, the bioconversion is highly dependent on the conditions, and we have not succeeded in repeating the process.

There are various possibilities to add a hydroxyl group at the C-14 of the intact epidaunorubicin, analogously to synthesis of doxorubicin from daunorubicin.

Purification of epirubicin is carried out by chromatography and/or by crystallization after extraction of epirubicin from the synthesis mixture. However, to achieve the quality requested for active pharmaceutical ingredient, chromatography separation to give epirubicin in ≥ 97 % purity is essential.

### Conversion of ε-rhodomycinone to idarubicin

It is known that biosynthesis proceeds in the sequence shown in Figure 1. Aklavinone, a typical precursor for several anthracyclines, is 11-hydroxylated to form ε-rhodomycinone, which is glycosylated. The modifications in the position 10 need for a glycosylated form even though other sugar residues, such as rhodosamine, are accepted substrates. After 10-modifications, 13-oxygenation takes place and the ultimate step for daunorubicin biosynthesis is an O-methylation at C-4. Therefore, 10- and 13-modifications as well as glycosylation are successful despite the 4-deoxy-form. Both 4-deoxyaklavinone and 4-deoxy-ε-rhodomycinone are converted into idarubicin.

The process of the present invention is indeed efficient since it allows production and recovery of ε-rhodomycinone, epidaunorubicin, 13 DHED and 4'-epi-feudomycin from a single fermentation batch.

For the reasons listed above it is advantageous to use a strain according to the present invention in fermentation to produce epirubicin and idarubicin for commercial use. The process according to the present invention enables the production of the important anthracyclines in a high yield with low costs. The diagram of the fermentation process is described in Figure 2.

A more detailed description of the present invention is given in the examples below.

It will be obvious to a person skilled in the art that, as the technology advances, the inventive concept can be implemented in various ways. The invention and its embodiments are not limited to the examples described above but may vary within the scope of the claims.

### EXAMPLES

### Example 1.

### Construction of microbial strains producing high titres of epidaunomycins and ε-rhodomycinone

Manipulation of the *Streptomyces* DNA was performed in *E. coli* and propagated DNA was then introduced into *Streptomyces* strains. The bacterial strains and plasmids used in this work are described in Table 3 below.

**Table 3. Bacterial strains and plasmids.**

| **Strain/plasmid** | **Description** | **Reference or Source** |
|---|---|---|
| *E. coli* XL1-Blue | *E. coli* cloning host | Stratagene |
| G001 | Daunomycin producing *Streptomyces* strain | DSM12245 |
| G001/pB70dv | Epidaunomycins and epi-carminomycins producing *Streptomyces* strain | DSM 19076 |

Cultivations of *Streptomyces* and *E*. *coli* strains as well as isolations and manipulations of DNA were carried out as described in the laboratory manuals of Hopwood et al. (1985) and Sambrook et al. (1989), respectively. Plasmids were introduced into *E. coli* strains by high-voltage electroporation and into *Streptomyces* by protoplast transformation (Ylihonko et al., 1996).

The gene *snog*C was used as a probe for cloning of corresponding genes from streptomycetes gene libraries. The genes cloned and propagated in *E. coli* were transferred into G001 in a vector pIJE486 and products obtained in small scale cultivations were studied by comparing to G001 products. In this way the clone carrying *ekr8* was found to produce epidaunomycins in addition to daunomycins. The clone was selected that was unable to produce typical daunorubicin metabolites whereas accumulating epidaunomycins and it was designated as G001/pB70dv (deposit no: DSM 19076). Products obtained in cultivating the clone in conditions as described in Example 2, revealed both epidaunomycins and epi-carminomycins.

The plasmid containing clone G001/pB70dv was cultivated several rounds in TSB-medium (Oxoid Tryptone Soya Broth powder 30 g per 1 litre) to drop out the plasmid. The strain obtained did not carry the plasmid and produced aglycones. The gene *ekr8* was cloned into different constructs carrying the genes involved in late steps of anthracycline biosynthesis and introduced into aglycone producing strain mentioned above. Analysis of samples from small scale cultivations in conditions described in example 2 were studied and the clone obtained carrying the plasmid pB89rdmB was able to produce epidaunomycin metabolites whereas epi-carminomycins were no more accumulated in the culture broth. The quantities are shown in Table 2 above.

The production of epi-carminomycins may be the result of incomplete activity of 4-O-methylase in the clone G001/pB70dv. Therefore, the gene rdmB which was cloned in the construction pB89rdmB was suggested to be responsible for complete biosynthesis to avoid epi-carminomycins. *rdmB* (ACCESSION U10405) show high sequence similarity to *dnrK* even though O-methyl transferase activity has not been demonstrated (see Jansson et al. 2003).

The strain carrying pB89rdmB was further mutagenized by NTG (N-methyl-N'-nitro-N-nitrosoguanidine) (Sigma-Aldrich) Roughly 200 colonies were picked up on agar plates after mutagenization and first cultivated in 3 ml of E1-medium. For further details of cultivation conditions, see example 2 below. The clones obtained by random mutagenization with improved epidaunomycins production and identical product profile to that of the pB89rdmB carrying strain were cultivated several times. Microbial strains were finally selected, that were able to produce epidaunomycin metabolites more than the pB89rdmB carrying clone in same proportions and without thiostrepton in medium for selection pressure.

Stability of such strains for suitability to commercial production was demonstrated by fourteen repeated cultivations (data not shown).

### Example 2.

### Culturing and product profile of microbial strains producing high titres of epidaunomycins and ε-rhodomycinone

Strains, obtained as described in example 1, were cultivated in 50 ml of E1-medium supplemented with XAD-7 (15 g/l). (E1: Per litre of tap water: glucose 20 g; soluble starch 20 g; Peptide 5 g; Yeast extract 2.5 g; K₂HPO₄•3H₂O 1.3 g; MgSO₄•7H₂0 1 g; NaCl 3 g; CaCO₃ 3 g; pH 7-7.5).

To determine the production titre the compounds were extracted from the fourth growth day until the tenth day. To determine the amount of anthracycline metabolites produced, XAD-7 was decanted with water from one cultivation flask and washed XAD-7 was extracted with 40 ml of methanol shaking for at least 30 min. The HPLC was used for analysing the samples. The use of XAD-7 in E1-medium increased the epidaunomycin yield to 500-700 mg/l from about 400 - 500 mg/l. See other products on Table 2. The optimum production time was eleven days. The changes in colony morphology were not found at the end of cultivation in the presence of the adsorbent, suggesting that it is possible to prevent the toxic and mutagen effect of epidaunomycin metabolites by adsorbing those into XAD during cultivation.

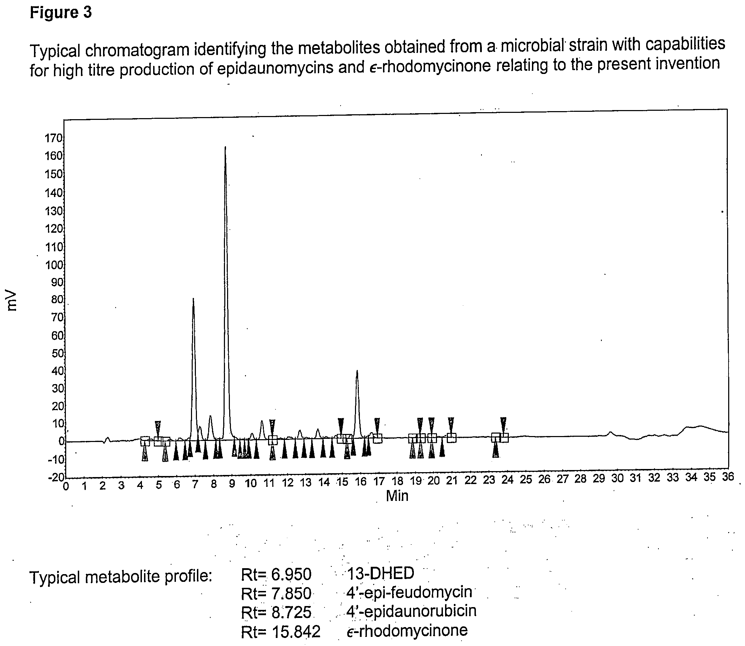

A typical chromatogram of the products obtained is shown in Figure 3.

### Example 3.

### Production of epidaunomycin metabolites and ε-rhodomycinone in fermentation

Seed culture was made by cultivating a strain with the capability of high titre production of epidaunomycins and ε-rhodomycinone in four flasks with 60 ml of the E1 medium supplemented with thiostrepton (5 mg/l) for four days. The cultures were combined and the 240 ml of the culture broth was used to inoculate a 20 I E1-medium supplemented with XAD-7 (15 g/l) in the fermentor. Fermentation was carried out in 20 litres volume for 11 days at the temperature of 30 °C and 34°C, 350 rpm with the aeration of 10 l/min.

### Example 4.

### Recovery and purification of crude epidaunomycins and ε-rhodomycinone.

### 4.1. Purification of crude epidaunomycins

The substituents containing epidaunorubicin and ε-rhodomycinone adsorbed to XAD-7 were decanted from the 20 litre culture broth obtained from fermentation. The resin was washed to remove cell debris by water. Pellet was extracted with 1 litre of methanol for two to five times.

The aglycones were extracted with chloroform by adding 1 l of chloroform to the 3 litre of combined methanol extracts. Solvent and water layers were separated. Glycosides in water phase were extracted to chloroform at lightly alkaline pH and pH was stabilized with saturated Na-HCO₃. Salts were removed by washing with water. Finally the chloroform-phase is filtrated through a cartridge filter. Yield of epidaunomycin using the said process corresponded to the 70 % of the crude extract.

### 4.2 Purification of ε-rhodomycinone

The solvent fraction obtained from extraction in the paragraph 4.1. above was dried and concentrated to small volume. Flash chromatography was done using chloroform as an eluent. The purified fraction was crystallized by dissolving into chloroform-methanol mixture and concentrated to small volume and > 90 % purity of ε-rhodomycinone was obtained. Dried precipitate was dissolved and purification was done by flash-chromatography using a solvent system with CHCl₃:Acetone:Methanol. Glycosides are extracted. This is done twice to maximize the yield.

After extracting all the glycosides to chloroform, the pH is stabilized by extracting with saturated NaHCO₃. Then salts are removed by extracting with RO-water for two to four times. The phases are separated with a two-phase separator.

### Example 5.

### Separation of individual compounds from epidaunomycins fraction

To separate epidaunorubicin, 13-DHED and epi-feudomycin from epidaunomycins obtained in Example 4.1, chromatography was carried out. The filtrated chloroform is pumped into a silica column and purified by chromatography using chloroform-methanol-solution as a mobile phase.

Pure fractions of each three metabolites were collected, and fractions of each product were pooled. To epidaunorubicin fractions, butanol was added and acidic pH was adjusted. After that the evaporation was started. When the evaporation was continued epidaunorubicin starts to crystallize. The crystals are filtrated and dried in a vacuum cabinet. The fractions of 13-DHED were crystallized by ethanol-water solutions.

### Example 6.

### Synthetic conversion of crude epidaunomycin into epirubicin

Purified epidaunorubicin was converted into epirubicin by chemical synthesis as a one-pot reaction consisting of two reactions; bromination and hydrolysis. In the first one C-14-position is substituted with bromine and in the second step C-14 is hydroxylated leading to formation of epirubicin. Epidauno-rubicin obtained as described in Example 5 was dissolved in methanol and bromine was added. Reaction was done under 10°C and analyzed by HPLC. The bromination reaction was stopped after one day by addition of sodiumformiate buffer. pH was adjusted < 3 and the temperature of the mixture is adjusted to 50-60°C. Reaction was monitored with HPLC and when the amount of epirubicin does not get higher anymore the reaction was stopped by cooling down the reaction mixture.

Purification was done by RP-silica chromatography. Fractions were collected and analyzed with HPLC and pure fractions (purity >97 %) were pooled. After crystallization the purity exceeding 98 % was obtained.

### Example 7.

### Analytical measurement of aglycones and anthracyclines

### HPLC:

*Equipment: Jasco HPLC.*
*Column: Phenomenex, Aqua, 150 x 4. 6 mm, 3 µm*
*Solvent A: 0.05 % TEA (pH=2.0 with TFA)*
*Solvent B:10% MeOH in THF*
*Temperature of the column: room temperature*
*Stream velocity: 1 ml*/*min*
*Detection: UV-Vis, 480 nm*
*Injection volume: 5 µl*

**Gradient:**

| ***time (min)*** | ***Solvent A [%]*** | ***Solvent B [%]*** |
|---|---|---|
| *0* | *80* | *20* |
| *10* | *60* | *40* |
| *27* | *45* | *55* |
| *28* | *10* | *90* |
| *30* | *10* | *90* |
| *31* | *80* | *20* |
| *36* | *80* | *20* |

### Deposited microorganisms

The following microorganism was deposited under the rules of the Budapest Treaty at DMSZ-Deutsche Sammlung von Mikroorganismen und Zellkulturen GmbH, Mascheroder Weg 1 b, D-38124 Braunschweig, Germany

| Microorganism | Accession number | Date of deposit |
|---|---|---|
| G001/pB70dV | DSM 19076 | 2007-02-23 |

### List of References

Di Marco A, Silvestrini R, Gaetani M, Soldati M, Orezzi P, Dasdia T, Scarpinato BM, Valentini L: 1964, 'daunomycin' , a new antibiotic of the rhodomycin group. Nature 15: 706-707.
Hopwood DA, Bibb MJ, Chater KF, Kieser T, Bruton CJ, Kieser HM, Lydiate DJ, Smith CP, Ward JM and Schrempf H (1985) Genetic Manipulation of Streptomyces: a Laboratory Manual. John Innes Foundation, Norwich.
Jansson A, Niemi J, Lindqvist Y, Mantsala P and Schneider G (2003) Crystal structure of aclacinomycin-10-hydroxylase, a S-adenosyl-L-methionine-dependent methyltransferase homolog involved in anthracycline biosynthesis in Streptomyces purpurascens. J Mol Biol 334:269-280.
Metsä-Ketelä M, Palmu K, Kunnari T, Ylihonko K and Mäntsälä P (2003) Engineering Anthracycline Biosynthesis toward Angucyclines. Antimicrobial Agents and Chemotherapy, 47 (4), 1291-1296
Niemi J, Kantola J, Ylihonko K, Mäntsälä, P. (2002) Anthracycline biosynthesis: stps, enzymes and genes (review) in Microbial secondary metabolites: biosynthesis, genetics and regulation (eds. Francisco Fierro & Juan Francisco Martin). Research Signpost 37/661, Kerala, India.
Otten SL, Gallo MA, Madduri K, Liu X, Hutchinson CR. (1997) Cloning and characterization of the Streptomyces peucetius dnmZUV genes encoding three enzymes required for biosynthesis of the daunorubicin precursor thymidine diphospho-L-daunosamine. J Bacteriol. 179 (13), 4446-50.
Sambrook J, Fritsch EF and Maniatis T (1989) Molecular Cloning: a Laboratory Manual. Cold Spring Harbor Laboratory, Cold Spring Harbor, NY.
Torkkell S, Kunnari T, Palmu K, Mäntsälä P, Hakala J and Ylihonko K (2001) The entire nogalamycin biosynthetic gene cluster of Streptomyces nogalater. characterization of a 20-kb DNA region and generation of hybrid structures. Molecular Genetics and Genomics 266:276-288.
Torkkell S. 2001, Anthracycline antibiotics: Biosynthetic pathway and molecular genetics of nogalamycin, a product of Streptomyces nogalater. Publications in Annales Universitatis Turkuensis-series nr: 275.
Ylihonko K, Tuikkanen J, Jussila S, Cong L and Mäntsälä P (1996) A gene cluster involved in nogalamycin biosynthesis from Streptomyces nogalater: sequence analysis and complementation of early blocked mutations of the anthracycline pathway. Mol Gen Genet 251:113-120

## Claims

1. A microbial strain of the species *Streptomyces peucetius var. caesius*, which produces anthracycline metabolites at a titre of at least 0.5 g/l, wherein the strain is producing at least 0.1 g/l fermentation broth of each of the compounds epidaunorubicin, 13-dihydro-epidaunorubicin, 4'-epi-feudomycin and ε-rhodomycinone, said strain being obtainable by (i) providing the *Streptomyces peucetius var.* caesius strain G001/pB70dv, deposited under the number DSM 19076, (ii) dropping out the plasmid from said strain by cultivating said strain several rounds in TSB-medium (30 g Oxoid Tryptone Soya Broth powder per 1 litre), and (iii) transforming said strain with a plasmid containing genes ekr8 and rdmB .

2. The strain according to claim 1, wherein this strain is producing of any of the compounds epidaunorubicin, 13-dihydro-epidaunorubicin, 4'-epi-feudomycin and ε-rhodomycinone at least 10 % of the total anthracycline metabolite fraction.

3. The strain according to claim 1 or 2, wherein the biosynthesis of endogeneous daunomycin metabolites is blocked.

4. The strain according to any of claims 1 - 3, wherein said baumycin production is blocked by random mutagenization.

5. A process for producing anthracycline metabolites by fermentation using a microbial producer strain according to any one of claims 1 - 4.

6. The process according to claim 5, wherein said anthracycline metabolites are selected from the group consisting of epidaunomycins and ε-rhodomycinone.

7. The process according to any one of claims 5 and 6, wherein said anthracycline metabolites are preferably selected from the group consisting of epidaunorubicin, 13-dihydroepidaunorubicin, 4'-epi-feudomcyin and ε-rhodomycinone.

8. The process according to any one of claims 5 - 7, wherein crude epidaunomycins and ε-rhodomycinone are absorbed from the fermentation broth by adding a resin at any time.

9. The process according to claim 8, wherein said resin is selected from the group of ionic and non-ionic adsorbents.

10. The process according to claim 9, wherein said resin is selected from the group of polystyrenes.

11. The process according to claim 10, wherein said resin is selected from the group consisting of XAD-7 and Diaion HP-20.

12. The process according to any one of claims 8 - 11, wherein said resin is added in an amount of 1 - 100 g/l.

13. The process according to claim 12, wherein said resin is preferably added in an amount of 15 - 40 g/l.

## Patentansprüche

1. Mikrobieller Stamm der Spezies *Streptomyces peucetius var. caesius*, der Anthracyclinmetabolite mit einem Titer von wenigstens 0,5 g/l produziert, wobei der Stamm bei wenigstens 0,1 g/l Fermentationsbrühe jede der Verbindungen Epidaunorubicin, 13-Dihydroepidaunorubicin, 4'-Epifeudomycin und ε-Rhodomycinon produziert, wobei der Stamm erhältlich ist durch
(i) Bereitstellen des *Streptomyces-peucetius-var.caesius-*Stamms G001/pB70dv, hinterlegt unter der Nummer DSM 19076,
(ii) Dropping-out des Plasmids aus dem Stamm durch Kultivieren des Stamms mehrerer Runden in TSB-Medium (30 g Oxoid-Trypton-Sojabrühe-Pulver pro 1 Liter) und (iii) Transformieren des Stamms mit einem Plasmid, das die Gene ekr8 und rdmB enthält.

2. Stamm gemäß Anspruch 1, wobei dieser Stamm eine beliebige der Verbindungen Epidaunorubicin, 13-Dihydroepidaunorubicin, 4'-Epifeudomycin und ε-Rhodomycinon mit wenigstens 10% der gesamten Anthracyclinmetaboliten-Fraktion produziert.

3. Stamm gemäß Anspruch 1 oder 2, wobei die Biosynthese von endogenem Daunomcyinmetaboliten blockiert ist.

4. Stamm gemäß einem der Ansprüche 1-3, wobei die Baumycin-Produktion durch zufallsbestimmte Mutagenisierung blockiert ist.

5. Verfahren zur Herstellung von Anthracyclinmetaboliten durch Fermentation unter Verwendung eines mikrobiellen Produzentenstamms gemäß einem der Ansprüche 1 bis 4.

6. Verfahren gemäß Anspruch 5, wobei die Anthracyclinmetaboliten aus der Gruppe, bestehend aus Epidaunomycinen und ε-Rhodomycinon, ausgewählt werden.

7. Verfahren gemäß einem der Ansprüche 5 und 6, wobei die Anthracyclinmetaboliten vorzugsweise aus der Gruppe, bestehend aus Epidaunorubicin, 13-Dihydroepidaunorubicin, 4'-Epifeudomycin und ε-Rhodomycinon, ausgewählt werden.

8. Verfahren gemäß einem der Ansprüche 5 bis 7, wobei rohe Epidaunomycine und ε-Rhodomycinon aus der Fermentationsbrühe durch Zusetzen eines Harzes zu einer beliebigen Zeit absorbiert werden.

9. Verfahren gemäß Anspruch 8, wobei das Harz aus der Gruppe ionischer und nicht-ionischer Adsorbentien ausgewählt wird.

10. Verfahren gemäß Anspruch 9, wobei das Harz aus der Gruppe von Polystyrolen ausgewählt wird.

11. Verfahren gemäß Anspruch 10, wobei das Harz aus der Gruppe, bestehend aus XAD-7 und Diaion-HP-20, ausgewählt wird.

12. Verfahren gemäß einem der Ansprüche 8-11, wobei das Harz in einer Menge von 1-100 g/l zugesetzt wird.

13. Verfahren gemäß Anspruch 12, wobei das Harz vorzugsweise in einer Menge von 15-40 g/l zugesetzt wird.

## Revendications

1. Souche microbienne de l'espèce *Streptomyces peucetius var. caesius*, qui produit des métabolites d'anthracycline à un titre d'au moins 0,5 g/L, dans laquelle la souche produit au moins 0,1 g/L de bouillon de fermentation de chacun des composés épidaunorubicine, 13-dihydro-épidaunorubicine, 4'-épi-feudomycine et ε-rhodomycinone, ladite souche pouvant être obtenue par (i) fourniture de la souche G001/pB70dv de *Streptomyces peucetius var. caesius*, déposée sous le numéro DSM 19076, (ii) abandon du plasmide issu de ladite souche par culture de ladite souche pendant plusieurs passages dans le milieu TSB (30 g de poudre de bouillon soja Tryptone Oxoid pour 1 litre), et (iii) transformation de ladite souche avec un plasmide contenant les gènes ekr8 et rdmB.

2. Souche selon la revendication 1, dans laquelle cette souche produit l'un quelconque des composés épidaunorubicine, 13-dihydro-épidaunorubicine, 4'-épi-feudomycine et ε-rhodomycinone à au moins 10 % de la fraction de métabolite d'anthracycline totale.

3. Souche selon la revendication 1 ou 2, dans laquelle la biosynthèse de métabolites de daunomycine endogènes est bloquée.

4. Souche selon l'une quelconque des revendications 1 à 3, dans laquelle ladite production de baumycine est bloquée par mutagénisation aléatoire.

5. Procédé de production de métabolites d'anthracycline par fermentation utilisant une souche productrice microbienne selon l'une quelconque des revendications 1 à 4.

6. Procédé selon la revendication 5, dans lequel lesdits métabolites d'anthracycline sont choisis dans le groupe consistant en les épidaunomycines et l'ε-rhodomycinone.

7. Procédé selon l'une quelconque des revendications 5 et 6, dans lequel lesdits métabolites d'anthracycline sont de préférence choisis dans le groupe consistant en l'épidaunorubicine, la 13-dihydroépidaunorubicine, la 4'-épi-feudomycine et l'ε-rhodomycinone.

8. Procédé selon l'une quelconque des revendications 5 à 7, dans lequel des épidaunomycines et l'ε-rhodomycinone brutes sont absorbées du bouillon de fermentation par addition d'une résine à chaque instant.

9. Procédé selon la revendication 8, dans lequel ladite résine est choisie dans le groupe des adsorbants ioniques et non ioniques.

10. Procédé selon la revendication 9, dans lequel ladite résine est choisie dans le groupe des polystyrènes.

11. Procédé selon la revendication 10, dans lequel ladite résine est choisie dans le groupe consistant en XAD-7 et Diaion HP-20.

12. Procédé selon l'une quelconque des revendications 8 à 11, dans lequel ladite résine est ajoutée dans une quantité de 1 à 100 g/L.

13. Procédé selon la revendication 12, dans lequel ladite résine est de préférence ajoutée dans une quantité de 15 à 40 g/L.
